# EUROPEAN PATENT APPLICATION

(11) **EP 1 837 788 A1**
(43) Date of publication of application: **26.09.2007**
(21) Application number: 07005306.1
(22) Date of filing: 14.03.2007
(51) Int. Cl.: G06F 19/00

(54) **Data management system, data transmitter/receiver, data management server, and data management method**

(30) Priority: 19.03.2006 JP 2006075764
(71) Applicant: TANITA CORPORATION, Tokyo 174-8630 (JP)
(72) Inventor: Hasegawa, Hiroki, Tokyo 174-8630 (JP)
(74) Representative: Müller-Boré & Partner Patentanwälte

(57) **Abstract**

To provide a data management system or the like by which a distance between a data measuring instrument 14 or the like, e.g., a body composition monitor and a PC 11 is not restricted to a wire cable, a troublesome operation, e.g., attachment/detachment of a memory medium for data exchange is not required, input of measurement data, e.g., a health testing data does not take trouble, and a device structure is inexpensive.

When a communication button 21 is pushed, a relay key 12 transmits predetermined information to a data measuring instrument 14 or the like. The data measuring instrument 14 or the like transmit measurement data to the relay key 12 side. The relay key 12 transmits to the PC 11 measurement information based on the received measurement data. The PC 11 transmits to a provider side server 44 the measurement information transmitted from the relay key 12. The provider side server 44 records the transmitted measurement information in a database DB 46 based on a predetermined format, and allows the PC 11 side to browse the measurement information recorded in the database DB 46.

## Description

### BACKGROUND OF THE INVENTION

### Field of the Invention

The present invention relates to a data management system or the like including : an information terminal device and a data management server connected with each other through a network; a data transmitter-receiver detachably connected with the information terminal device through a predetermined interface; and a data measuring instrument connected with the data transmitter-receiver based on a predetermined wireless communication system.

### Description of the Related Art

In recent years, systems that connect a body composition monitor or the like to a personal computer (PC) to conduct health care (or health management) have been developed. As such systems, one that connects a body composition monitor or the like with a PC through a wire cable forms a mainstream (see "products catalog", <URL:http://www.healthcare.omron.co.jp/product/hbf354it_2.html>), but one that fetches measurement data to the PC through a detachable memory medium is also present. A system that utilizes the Internet to conduct health care has been also developed, and measurement data like health testing data or the like received from e.g., a medical institution is input in such a system (see "the Internet health care Kentaro", <URL:http://www.kenta.ne.jp/sa-bisumenu02.htm>). Considering remote medical care, a large-scale system configuration is generally required (see "What is a remote medical care", <URL:http://tele00.suzuka-u.ac.jp/!medicine/telemedicine.htm>).

As explained above, since a body composition monitor or the like is connected with a PC through a wire cable in a conventional health care system, a distance between the body composition monitor or the like and the PC is restricted to a length of the wire cable, and there was a problem that measurement cannot be performed at a desired position distanced from the PC. In case of a system that fetches measurement data to a PC from a body composition monitor or the like through a detachable memory medium, there was a problem that a troublesome operation, i.e., attachment/detachment of the memory medium for data exchange is required. In case of a system that utilizes the Internet to conduct health care, there was a problem that inputting measurement data of health-testing data or the like took trouble. In case of a remote medical, there was a problem that devices or the like were very expensive.

### SUMMARY OF THE INVENTION

Therefore, the present invention has been achieved to solve the problems described above and it is an object of the present invention to provide a data management system or the like by which a distance between a data measuring instrument of a body composition monitor or the like and a PC is not restricted to a length of a wire cable, a troublesome operation, i.e., attachment/detachment of a memory medium for data exchange is not required, inputting measurement data of health testing data or the like does not take trouble, and a device configuration is inexpensive.

According to a first aspect of the present invention, there is provided a data management system comprising: an information terminal device and a data management server that are connected with each other through a network; a data transmitter-receiver detachably connected with the information terminal device through a predetermined interface; and a data measuring instrument connected with the data transmitter-receiver based on a predetermined wireless communication system, wherein the data transmitter-receiver comprises: information transmitting means for transmitting predetermined information to the data measuring instrument; measurement data receiving means for receiving measurement data of a user transmitted from the data measuring instrument based on predetermined information transmitted by the information transmitting means; and measurement information transmitting means for transmitting measurement information based on the measurement data received by the measurement data receiving means to the information terminal device, the information terminal device comprises: network transmitting means for transmitting the measurement information transmitted by the measurement information transmitting means to the data management server, and the data management server comprises: measurement information recording means for recording the measurement information transmitted by the network transmitting means in a data recording section in accordance with each user based on a predetermined format.

According to a second aspect of the present invention, there is provided a data transmitter-receiver detachably connected with an information terminal device through a predetermined interface, comprising: information transmitting means for transmitting predetermined information to a data measuring instrument connected therewith based on a predetermined wireless communication system; measurement data receiving means for receiving measurement data of a user transmitted from the data measuring instrument based on the predetermined information transmitted by the information transmitting means; and measurement information transmitting means for transmitting measurement information based on the measurement data received by the measurement data receiving means to the information terminal device.

According to a third aspect of the present invention, there is provided a data management server connected with an information terminal device through a network, wherein a data transmitter-receiver detachably connected with the information terminal device through a predetermined interface transmits predetermined information to a data measuring instrument connected with the data transmitter-receiver based on a predetermined wireless communication system, measurement data of a user transmitted from the data measuring instrument is received based on the predetermined information, and measurement information based on the measurement data is transmitted to the data management server through the information terminal device, and the data management server comprises measurement information recording means for recording the transmitted measurement information in a data recording section in accordance with each user based on a predetermined format.

According to a fourth aspect of the present invention, there is provided a data management method using: an information terminal device and a data management server connected with each other through a network; a data transmitter-receiver detachably connected with the information terminal device through a predetermined interface; and a data measuring instrument connected with the data transmitter-receiver based on a predetermined wireless communication system, comprising: an information-transmitting step at which the data transmitter-receiver transmits predetermined information to the data measuring instrument; a measurement data receiving step at which the data transmitter-receiver receives measurement data of a user transmitted from the data measuring instrument based on the predetermined information transmitted in the information transmitting step; a measurement information transmitting step at which the data transmitter-receiver transmits measurement information based on the measurement data received in the measurement data receiving step to the information terminal device; a network transmitting step at which the information terminal device transmits the measurement information transmitted in the measurement information transmitting step to the data management server, and a measurement information recording step at which the data management server records the measurement information transmitted in the network transmitting step in a data recording section in accordance with each user based on a predetermined format.

The above and other objects, effects, features and advantages of the present invention will become more apparent from the following description of the embodiments thereof taken in conjunction with the accompanying drawings.

### BRIEF DESCRIPTION OF THE DRAWINGS

Fig. 1 shows a data management system 1 in a first embodiment according to the present invention.
Fig. 2 shows a user information table 60 recorded in the database DB 46.
Fig. 3 shows a structure of the relay key 12.
Fig. 4 shows a connection state between the relay key 12 and the PC 11.
Fig. 5 shows a detailed structure in the home system 10.
Fig. 6 shows a flow of a data management method in a first embodiment according to the present invention in the form of flowcharts.
Fig. 7 shows a data management system 2 in a second embodiment according to the present invention.
Fig. 8 shows an association table 80 recorded in the database DB 46.
Fig. 9 shows a flow of a data management method in a second embodiment according to the present invention in the form of a flowchart.
Figs. 10A and (B) show a user information table 60' or the like in a third embodiment according to the present invention.
Fig. 11 shows a user information table 60' or the like when measuring instrument registration of the electronic sphygmomanometer 16 and recording of blood pressure measurement data have been performed in relation to the user.
Fig. 12 shows a user information table 60' or the like when a plurality of types of measurement data are included like the body composition monitor 14.

### DETAILED DESCRIPTION OF THE PREFERRED EMBODIMENTS

Each embodiment will now be described in detail hereinafter with reference to the drawings. It is to be noted that the same or similar reference numerals are applied to the same or similar parts and elements throughout the drawings, and the description of the same or similar parts and elements will be omitted or simplified.

### First Embodiment

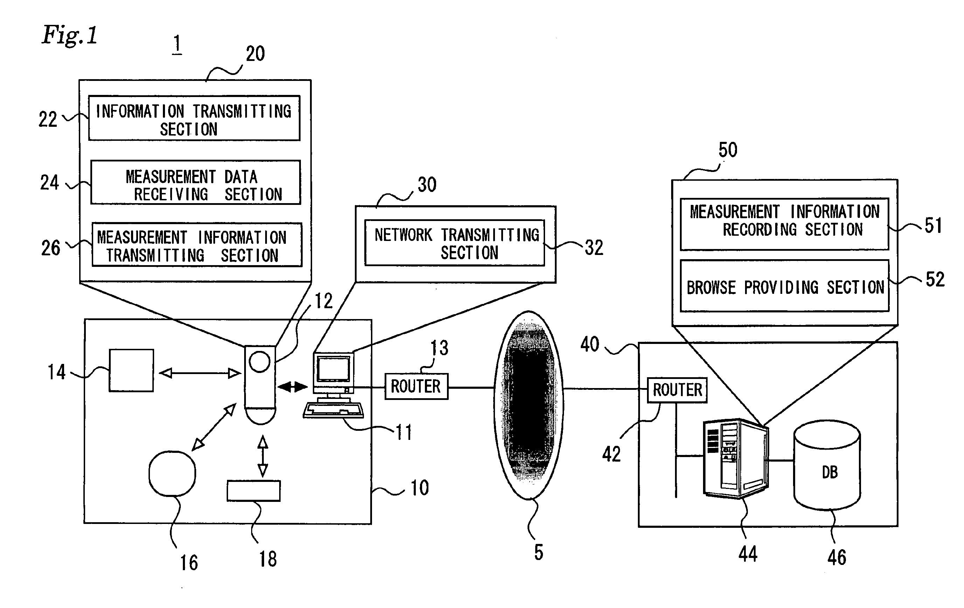

Fig. 1 shows a data management system 1 in a first embodiment according to the present invention. In Fig. 1, reference numeral 5 denotes a network, e.g., the Internet; 10, a home system of a measurement target person (not shown) that performs measurement to obtain health care data; 11, a PC (an information terminal device) connected with the network 5 through a router 13; 40, a health care provider side system that manages the health care data of the measurement target person; and 44, a server of the health care provider side system (a data management server which will be referred to as a "provider side server" hereinafter) connected with the network 5 through a router 42. As shown in Fig. 1, the PC 11 is connected with the provider side server 44 through the network 5.

In the home system 10 shown in Fig. 1, reference numeral 12 designates a relay key (a data transmitter-receiver) detachably connected with the PC 11 via a predetermined interface, and each of reference numerals 14, 16, and 18 denotes a data measuring instrument connected with the relay key 12 based on a predetermined wireless communication system (which will be explained later). As the predetermined interface, a USB (Universal Serial Bus) can be used, for example. It is needless to say that other arbitrary interfaces can be also utilized.

In Fig. 1, reference numeral 20 designates a function block showing functions of the relay key 12. As shown in Fig. 1, the relay key 12 includes an information transmitting section (information transmitting means) 22 that transmits predetermined information to the data measuring instrument 14 or the like, a measurement data receiving section (measurement data receiving means) 24 that receives measurement data transmitted from the data measuring instrument 14 or the like based on the predetermined information transmitted by the information transmitting section 22, and a measurement information transmitting section (measurement information transmitting means) 26 that transmits to the PC 11 measurement information based on the measurement data received by the measurement data receiving section 24. The predetermined information, the measurement data or the like are recorded in a memory (not shown) in the relay key 12. The above-explained functions can be realized by appropriately combining hardware and software. As shown in Fig. 1, the data measuring instrument 14 or the like are connected with the PC 11 through the relay key 12, and the measurement data measured by the data measuring instrument 14 or the like can be transferred to the PC 11 via the relay key 12. Therefore, since a distance between the data measuring instrument 14 or the like and the PC 11 is not restricted to a length of a wire cable that connects both the members with each other like the conventional system, the measurement target person can perform measurement at a desired position distanced from the PC 11. A troublesome operation, i.e., attachment/detachment of a memory medium from the data measuring instrument 14 or the like to the PC 11 for data exchange is not required. It is preferable to perform communication between the data measuring instrument 14 or the like and the relay key 12 based on a wireless communication system (a predetermined wireless communication system) using infrared rays or specified low power radio or the like. The specified low power radio means wireless transmission requiring no license that is short-distance wireless transmission having an antennal power of 10 mW or below (a radio equipment standard ARIBSTD-T67 for a specified low power radio station telemeter, tele-control, and data transmission). It is preferable to connect the relay key 12 with the PC 11 through an interface using the USB. Therefore, the measurement data measured by the data measuring instrument 14 or the like can be automatically transmitted to the PC 11 via the relay key 12 in real time. As a result, the measurement target person does not have to take trouble when inputting the measurement data. As a configuration in the home system 10, the PC 11, the relay key 12, the data-measuring instrument 14 or the like alone can suffice, thereby providing an inexpensive device configuration.

Communication or the like between the PC 11 and the provider side server 44 will now be explained. In Fig. 1, reference numeral 30 denotes a function block showing a function of the PC 11. As depicted in Fig. 1, the PC 11 includes a network transmitting section (network transmitting means) 32 that transmits measurement information transmitted from the measurement information transmitting section 26 to the provider side server 44.

In Fig. 1, reference numeral 50 designates a function block showing functions of the provider side server 44. As shown in Fig. 1, the provider side server 44 includes a measurement information recording section (measurement information recording means) 51 that records the measurement information transmitted by the network transmitting section 32 in a database DB (a data recording section) 46 in accordance with each user based on a predetermined format, and a browse providing section (browse providing means) 52 that allows the PC 11 side to browse the measurement information recorded in the database DB 46. The above-explained respective functions of the PC 11 and the provider side server 44 can be mainly realized by software.

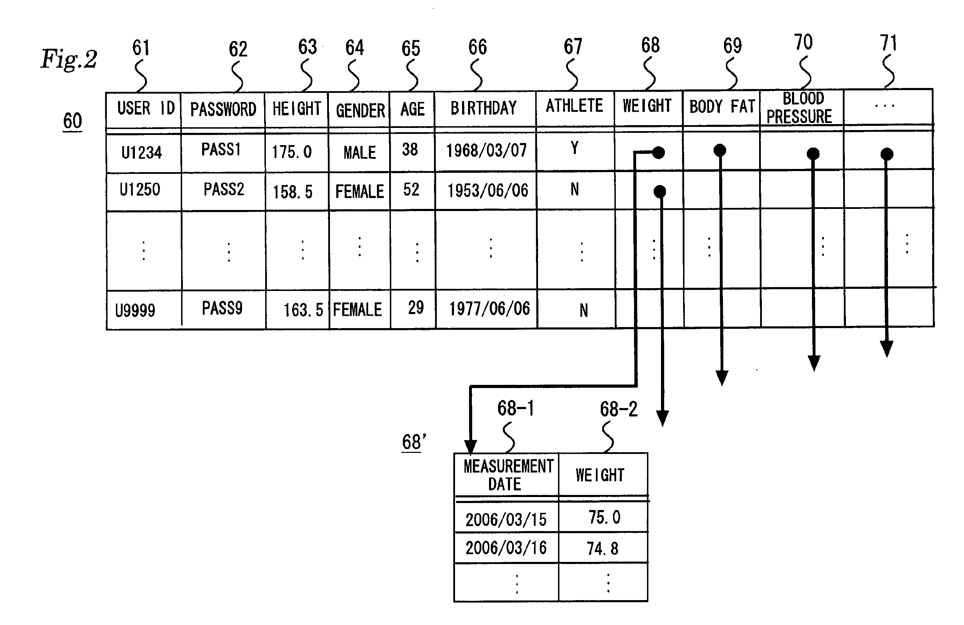

The predetermined format will now be described. Fig. 2 shows a user information table 60 recorded in the database DB 46. In Fig. 2, reference numeral 61 denotes a user (the measurement target person) ID section; 62, a password section; 63, a height section; 64, a gender section; 65, an age section; 66, a birthday section; and 67, an athlete section indicating an athlete or a standard. User information is information from the user ID section 61 to the athlete section 67. Of the user information, the user ID section 61 to the password section 62 are sections in which user authenticating information is recorded, and the height section 63 to the athlete section 67 are sections in which the user information excluding the user authenticating information is recorded. For example, in a first row of the user information table 60, U1234 is recorded in the user ID section 61; PASS1, the password section 62; 175.0 cm, the height section 63; male, in the gender section 64; 38, the age section 65; March 7, 1968, the birthday section 66; and Y (Yes), the athlete section 67. Although a user ID and a password are preferable as the user authenticating information as explained above, physical characteristics, e.g., fingerprint information, iris information, or a vein pattern or the like of a user that are utilized in biometrics authentication may be used. It is needless to say that one or more combinations of a user ID, a password, and physical characteristics of a user may be used. A user is subjected to membership registration to use the relay key 12 in a website of the health care provider side system 40, and records the user information in the database DB 46 in advance.

Measurement data is appropriately recorded in a weight section 68, a body fat section 69, a blood pressure section 70, a miscellaneous section 71, or the like in the user information table 60. In regard to the number (types) of the weight sections 68 or the like, a desired number of the sections can be provided in advance. An example of dynamically increasing the number of weight sections 68 or the like will be explained in conjunction with a third embodiment. As shown in Fig. 2, for example, it is good enough to record a pointer to a weight table 68' in the weight section 68 and sequentially record a measurement date and a measured weight in a measurement date section (which may include a time) 68-1 and a weight section 68-2 provided in the weight table 68'. The measurement date may be measured by using a timer in, e.g., the data-measuring instrument 14 or the like, the relay key 12, or the PC 11 and included in the measurement information. For example, in a first row of the weight table 68', 2006/03/15 (March 15, 2006) is recorded in the measurement date section 68-1, and 75.0 kg is recorded in the weight section 68-2. Alternatively, a list in which a measurement date and a measured weight form a pair ((a measurement date 1, a weight 1), (a measurement date 2, a weight 2), ...) or the like may be recorded in the weight section 68. Since information can be likewise recorded in the body fat section 69, the blood pressure section 70, the miscellaneous section 71 or the like of a given user and other information can be likewise recorded in the weight section 68 or the like of another use, an explanation of each section will be omitted.

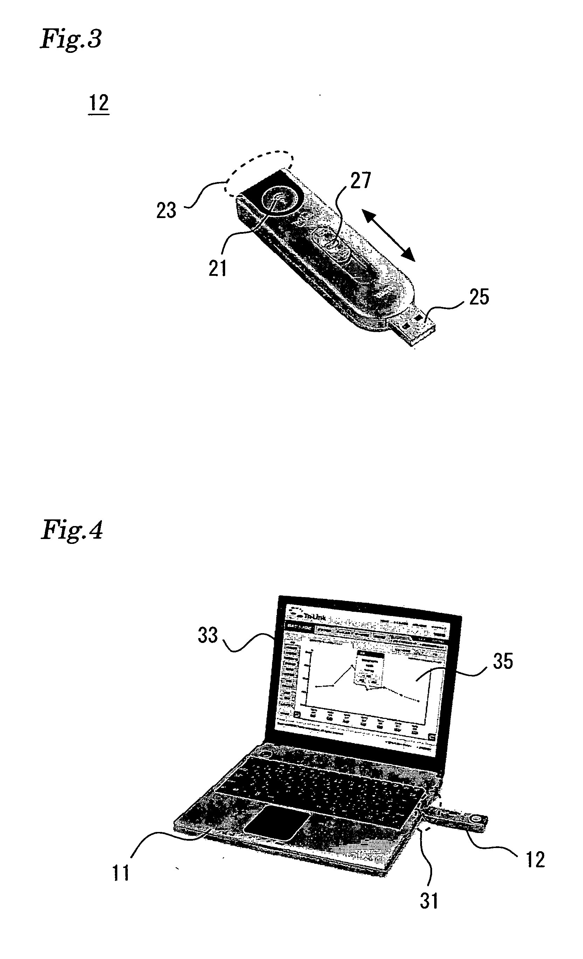

Fig. 3 shows a structure of the relay key 12. In Fig. 3, reference numeral 23 denotes a transmitter-receiver (surrounded by a dotted line) based on infrared rays or specified low power radio or the like that is utilized by the information transmitting section 22 to transmit predetermined information to the data measuring instrument 14 or the like. As the predetermined information, there are an activation command to active the data measuring instrument 14 or the like, individual setting information (the user information) such as a height or an age or the like of the measurement target person required by the data measuring instrument 14 or the like (e.g., a body composition monitor). The predetermined information is transmitted by pushing a communication button 21. When the predetermined information is transmitted to the data measuring instrument 14 or the like, the data measuring instrument 14 or the like are automatically activated based on the predetermined information to measure data, e.g., a weight and transmit measurement data of a user obtained from measurement to the relay key 12 side. The measurement data receiving section 24 receives the measurement data through an infrared transmitter-receiver 23 or the like. In Fig. 3, reference numeral 25 designates a USB terminal, and the USB terminal 25 can be pulled out of the relay key 12 or accommodated in the relay key 12 by sliding a slide key 27 in a direction of arrows depicted in Fig. 3. In a device using a conventional USB terminal, the USB terminal itself is always exposed to the outside of the device, and hence a cap must be put on the USB terminal for protection. Although the cap has a drawback that it can be readily lost, the cap is no longer necessary when the slide key 27 is provided in the relay key 12 according to the present invention.

Fig. 4 shows a connection state between the relay key 12 and the PC 11. As shown in Fig. 4, it is good enough to connect the USB terminal 25 (which is not displayed since it is connected in Fig. 4) of the relay key 12 with a USB port 31 of the PC 11. As explained above, the provider side server 44 includes the browse providing section 52 so that the browse providing section 52 allows the measurement information to be browsed in a display 33 of the PC 11 in various display formats 35, e.g., a graph or a table as shown in Fig. 4. For example, a change in a weight of a given user in accordance with a measurement date can be displayed in the form of a graph based on the weight table 68'. The browse providing section 52 may appropriately use the display formats 35 in accordance with a type of measurement data in the user information table 60.

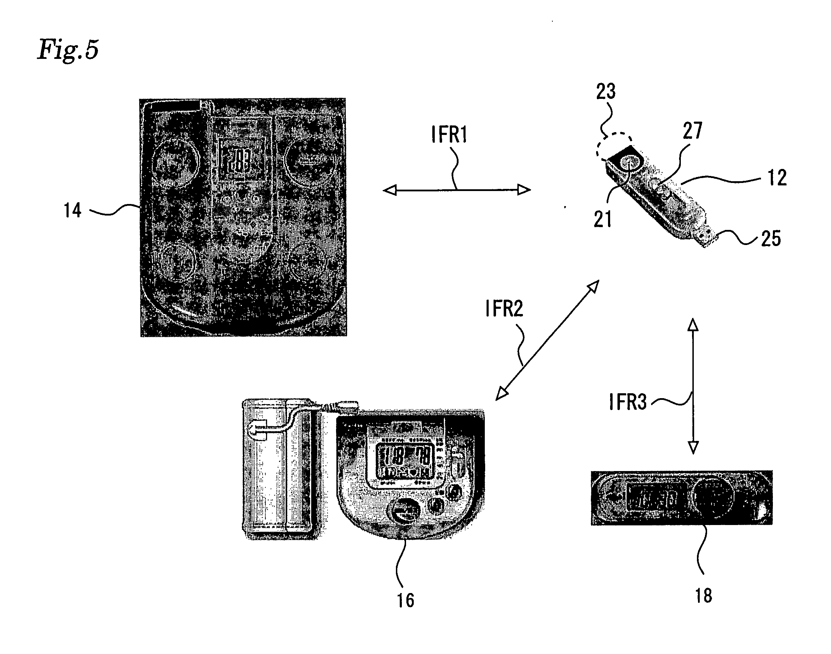

Fig. 5 shows a detailed configuration in the home system 10. In Fig. 5, parts denoted by the same reference numerals as those in Figs. 1 and 3 represent the same elements, thereby omitting an explanation thereof. As shown in Fig. 5, the data measuring instrument 14 is a body composition monitor; 16, an electronic sphygmomanometer; and 18, a pedometer. Although the relay key 12 is shown in the substantially same size as the other data measuring instrument 14 or the like in Fig. 5 for the convenience's sake of the drawing, it actually has a compact size substantially equal to a notebook. It is preferable for the data measuring instrument 14 or the like to be measuring instruments for data concerning a living body of the measurement target person, and they may be a urine gulcose meter or a thermometer or the like besides the data measuring instrument 14 or the like. Although Fig. 5 shows the three data measuring instrument 14 or the like for the convenience's sake, the number of the measuring instruments is not restricted to three, and a desired number of data measuring instruments can be set up. As a communication system between the data measuring instruments 14, 16, and 18 and the relay key 12, it is possible to adopt a transmission-receiving system based on infrared rays INFR1, INFR2, and INFR3, respectively. As explained above, the specified low power radio or the like may be used. In case of using the infrared rays, as a format of infrared transmission, using a general format of Association for Electric Home Appliances, an original format, or the like can suffice. For example, measurement data can be automatically transmitted to the relay key 12 by a simple operation, e.g., mounting the pedometer 18 on the relay key 12.

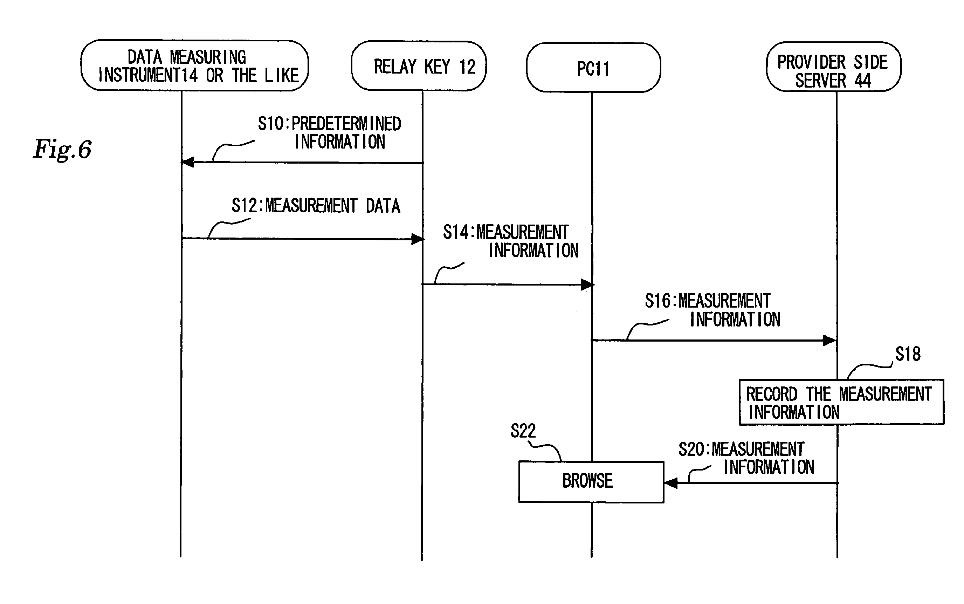

Fig. 6 shows a flow of a data management method in a first embodiment according to the present invention in the form of flowcharts. In Fig. 6, the leftmost flowchart shows a flow of processing in the data measuring instrument 14 or the like, the flowchart that is the second from the left shows a flow of processing on the relay key 12 side, the flowchart that is the second from the right shows a flow of processing on the PC 11 side, the rightmost flowchart shows a flow of processing in the provider side server 44, and a line connecting the respective flowcharts shows mutual communication. A detailed processing block will be omitted for the convenience's sake of the drawing. Although Fig. 6 does not show an end terminal in particular, it is assumed that processing explained below is appropriately repeated. As shown in Fig. 6, when a user first pushes the communication button 21 of the relay key 12, predetermined information is transmitted to the data measuring instrument 14 or the like (an information transmitting step: a step S10). The data measuring instrument 14 or the like are automatically activated based on the predetermined information as explained above to measure data of, e.g., a weight and transmit measurement data of the user obtained from measurement to the relay key 12 side. The relay key 12 receives the measurement data supplied from the data measuring instrument 14 or the like that were activated based on the predetermined information transmitted to the data measuring instrument 14 or the like at the information transmitting step (the step S10) and have performed measurement (a measurement data receiving step: a step S12). Subsequently, the relay key 12 transmits to the PC 11 measurement information based on the measurement data received at the measurement data receiving step (a step S12) (a measurement information transmitting step: a step S14).

The PC 11 transmits the measurement information transmitted at the measurement information transmitting step (the step S14) to the provider side server 44 (a network transmitting step: a step S16).

The provider side server 44 records the measurement information transmitted at the network transmitting step (the step S16) in the database DB 46 in accordance with each user based on a predetermined format (a measurement information recording step: a step S18). The PC 11 side is allowed to browse the measurement information recorded in the database DB 46 (the step S20, the step S22). A series operations, i.e., transmission of the measurement information from the PC 11 to the provider side server 44, recording of the measurement information in the database DB 46, and provision of browsing the measurement information to the PC 11 can be automatically carried out in a state where the relay key 12 is connected with the USB port 31 of the PC 11. In case of browsing the already transmitted measurement information later, it can be likewise performed in the state where the relay key 12 is connected with the USB port 31 of the PC 11.

As explained above, according to a first embodiment of the present invention, when a user pushes the communication button 21 of the relay key 12, the predetermined information is transmitted to the data measuring instrument 14 or the like. The data measuring instrument 14 or the like are automatically activated based on the predetermined information as explained above to measure data, e.g., a weight and transmit the measurement data obtained from measurement to the relay key 12 side. The relay key 12 receives the measurement data of the user supplied from the data measuring instrument 14 or the like that were activated based on the predetermined information transmitted to the data measuring instrument 14 or the like and have performed measurement. That is, just pushing the communication button 21 can acquire the measurement data of the user. The relay key 12 transmits the measurement information based on the received measurement data of the user to the PC 11. Since the data measuring instrument 14 or the like communicate with the relay key 12 based on the wireless communication system utilizing, e.g., infrared rays or the specified low power radio and the relay key 12 is connected with the PC 11 via the interface using the USB, the measurement data obtained from measurement by the data measuring instrument 14 or the like can be automatically transmitted to the PC 11 through the relay key 12 in real time. Therefore, the measurement target person does not have to take trouble when inputting the measurement data. As a configuration in the home system 10, only the PC 11, the relay 12, the data measuring instrument 14 or the like can suffice, thereby providing an inexpensive device structure.

Subsequently, the PC 11 transmits the measurement information supplied from the relay key 12 to the provider side server 44. The provider side server 44 records the transmitted measurement information in the database DB 46 in accordance with each user based on a predetermined format, and allows the PC 11 side to browse the measurement information recorded in the database DB 46. A series of operations, i.e., transmission of the measurement information from the PC 11 to the provider side server 44, recording of the measurement information in the database DB 46, and provision of browsing the measurement information to the PC 11 can be automatically carried out in a state where the relay key 12 is connected with the USB port 31 of the PC 11. As a result, it is possible to provide the data management system or the like by which a distance between the data measuring instrument 14 or the like, e.g., a body composition monitor and the PC 11 is not restricted to a length of a wire cable, a troublesome operation, e.g., attachment/detachment of a memory medium for data exchange is not required, inputting the measurement data, e.g., a health testing data does not take trouble, and an inexpensive device configuration is provided.

### Second Embodiment

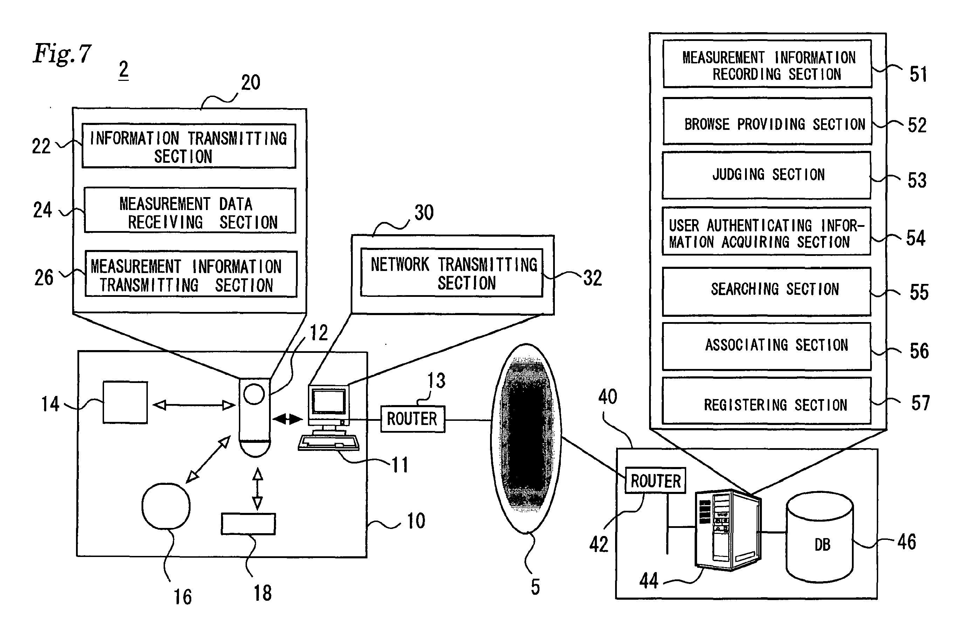

Fig. 7 shows a data management system 2 in a second embodiment according to the present invention. In Fig. 7, parts denoted by the same reference numerals as those in Fig. 1 represent the same elements/functions, thereby omitting an explanation thereof. A second embodiment is different from a first embodiment in that a function of before-use registration of the relay key 12 using user authenticating information is added. In more detail, as shown in a function block 50 in Fig. 7, a difference lies in that a judging section (judging means) 53, a user authenticating information acquiring section (user authenticating information acquiring means) 54, a searching or retrieving section (searching means) 55, an associating section (associating means) 56, and a registering section (registering means) 57 are added to the function of the provider side server 44.

The judging section 53 shown in Fig. 7 judges whether the relay key 12 has been registered before use based on user information including user authenticating information previously recorded in the database DB 46 and device authenticating information of the relay key 12 acquired through the PC 11. As the device authenticating information, a serial number recorded in a memory, e.g., an ROM in the relay key 12 is preferable. When a user inserts the relay key 12 into the USB port 31 of the PC 11 after membership registration, the provider side server 44 acquires a serial number through the PC 11. When the user information including a user ID or the like recorded in the database DB 46 in advance at the time of membership registration is not associated (which will be explained later) with the serial number of the relay key 12 acquired through the PC 11, the judging section 53 determines that the relay key 12 has not been registered before use. The judging section 53 can also confirm whether the serial number itself is adequate as a number of the relay key.

When the judging section 53 determines that the relay key 12 has not been registered before use, the user authenticating information acquiring section 54 acquires the user ID or the like via the PC 11. That is, the user again inputs the user ID or the like that have been input at the time of membership registration.

The searching section 55 searches or retrieves the database DB 46 based on the user ID or the like acquired by the user authenticating information acquiring section 54. Referring to Fig. 2, if the acquired user ID is U1234, it is possible to search a fact that this user ID or the like are recorded in the first row of the user information table 60.

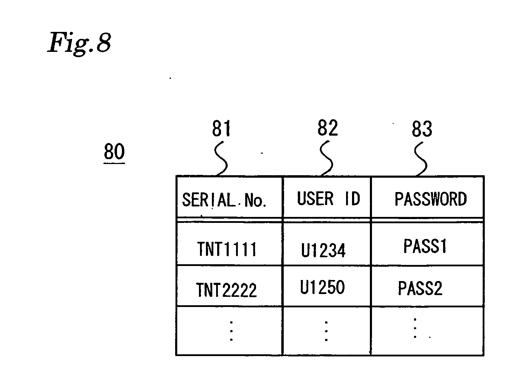

Fig. 8 shows an association table 80 recorded in the database DB 46. In Fig. 8, reference numeral 81 denotes a serial number (No.) section; 82, a user ID section; and 83, a password section. The associating section 56 associates the user ID or the like searched by the searching section 55 with a serial number by using the association table 80. For example, as shown in Fig. 2, when the user ID searched in the user information table 60 is U1234 and a password is PASS1, and a serial number is TNT1111, TNT1111 is recorded in the serial number section 81 in a given row of the association table 80 depicted in Fig. 8, and U1234 and PASS1 are respectively recorded in the user ID section 82 and the password section 83 in the same row, thereby performing association (string attachment). As a result, the serial number of the relay key 12 is registered in the database DB 46, and the user is associated with the relay key 12. Other arbitrary methods may be used for association and, for example, a pointer section in which a pointer to a row having a corresponding user ID in the user information table 60 may be provided in place of providing the user ID section 82 or the like in the association table 80.

The registering section 57 registers user information corresponding to the user ID or the like searched by the searching section 55 and excluding the user ID or the like in the relay key 12 side via the PC 11. That is, the user information from the height section 63 to the athlete section 67 of the user recorded in the user information table 60 shown in Fig. 2 is recorded (registered) in the relay key 12 side. As a result, before-use registration (initial registration) of the relay key 12 is completed.

Each authenticating information can be likewise used in transmission of the measurement data from the PC 11 to the provider side server 44, recording in the database DB 46 or the like explained in conjunction with a first embodiment. The measurement information transmitted by the measurement information transmitting section 26 includes the serial number of the relay key 12 in addition to the measurement data. Therefore, when the measurement information recording section 51 uses the association table 80 to determine that the serial number included in the measurement information transmitted by the network transmitting section 32 is associated with the user ID or the like, the measurement information is recorded in the user information table 60 in the database DB 46 in accordance with the user ID and other based on a predetermined format. As the predetermined format, the format of each section in the user information table 60 is preferable; other arbitrary formats may be used.

Likewise, each authenticating information can be used for provision of browsing from the provider side server 44 to the PC 11 explained in conjunction with a first embodiment. The browse providing section 52 requests the PC 11 side to input the user ID or the like, and allows the PC 11 side to browse the measurement information in a row of the user information table 60 corresponding to this user ID or the like when the input user ID or the like match with the user ID or the like recorded in the user information table 60 in the database DB 46. As seen from a user, just connecting the relay key 12 to the USB port 31 of the PC 11 allows jumping to a login screen of a member page in which the measurement data can be browsed.

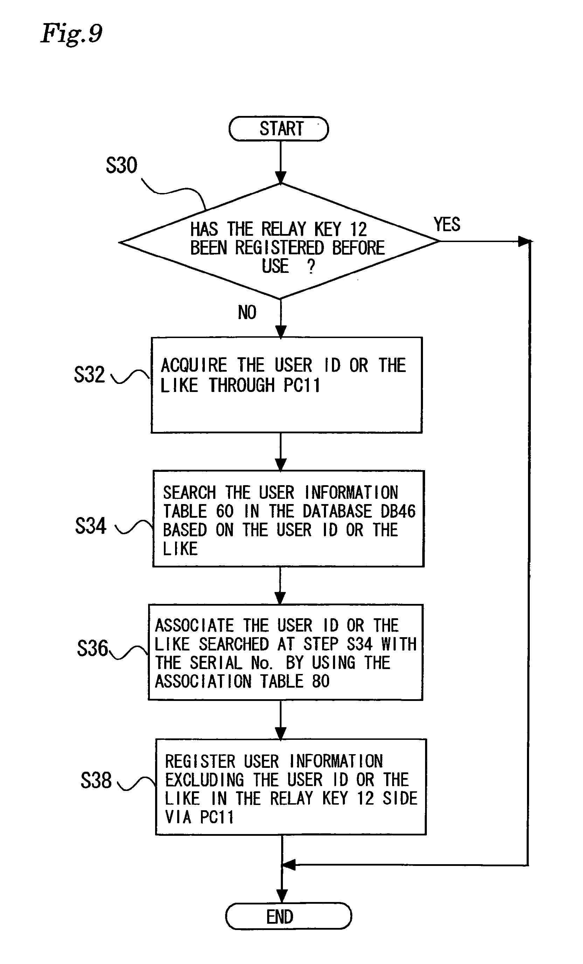

Fig. 9 shows a flow of a data management method in a second embodiment according to the present invention in the form of a flowchart. As shown in Fig. 9, the provider side server 44 judges whether the relay key 12 has been registered before use based on the user information including the user ID or the like previously recorded in the database DB 46 and the serial number of the relay key 12 acquired through the PC 11 (a judging step: a step S30). When it is determined that the relay key 12 has not been registered before use at the judging step (the step S30), the user ID or the like are acquired through the PC 11 (a user authenticating information acquiring step: a step S32). When it is determined that the relay key 12 has been registered before use at the judging step (the step S30), this flowchart is terminated. The user information table 60 in the database DB 46 is searched based on the user ID or the like acquired at the user authenticating information-acquiring step (the step S32) (a searching step: a step S34). The user ID or the like searched at the searching step (the step S34) are associated with the serial number by using the association table 80 (an associating step: a step S36). User information corresponding to the user ID or the like searched at the searching step (the step S34) and excluding the user ID or the like is registered in the relay key 12 side via the PC 11 (a registering step: a step S38).

As explained above, according to a second embodiment of the present invention, the user authenticating information and the function of before-use registration of the relay key 12 using this information can be added. As a result, in addition to the effects of a first embodiment, simple membership registration for a user can be performed, thereby improving protection of individual information of the user.

### Third Embodiment

In each of the foregoing embodiments, the number of the weight sections 68 or the like in the user information table 60 is a predetermined fixed number. In a third embodiment, an example where the weight sections 68 or the like are dynamically provided in accordance with addition or the like of the data measuring instruments will be explained. Measurement data transmitted from a data measuring instrument 14 or the like and received from a measurement data receiving section 24 can include measuring instrument identifying information (a measuring instrument ID) of the data measuring instrument 14 or the like. Therefore, the measuring instrument ID is included in measurement information transmitted by a network transmitting section 32. Recording this measuring instrument ID in a database DB 46 enables registration (measuring instrument registration) of the data measuring instrument 14 or the like to be used in accordance with each user. When it is determined that the measuring instrument ID of the data measuring instrument 14 or the like included in the measurement information transmitted by the network transmitting section 32 has not been registered in relation to a user corresponding to the measurement information, a measurement information recording section 51 subjects this measuring instrument ID to measuring instrument registration as a new ID, and records the measurement information in a data recording section in accordance with each user based on a predetermined format.

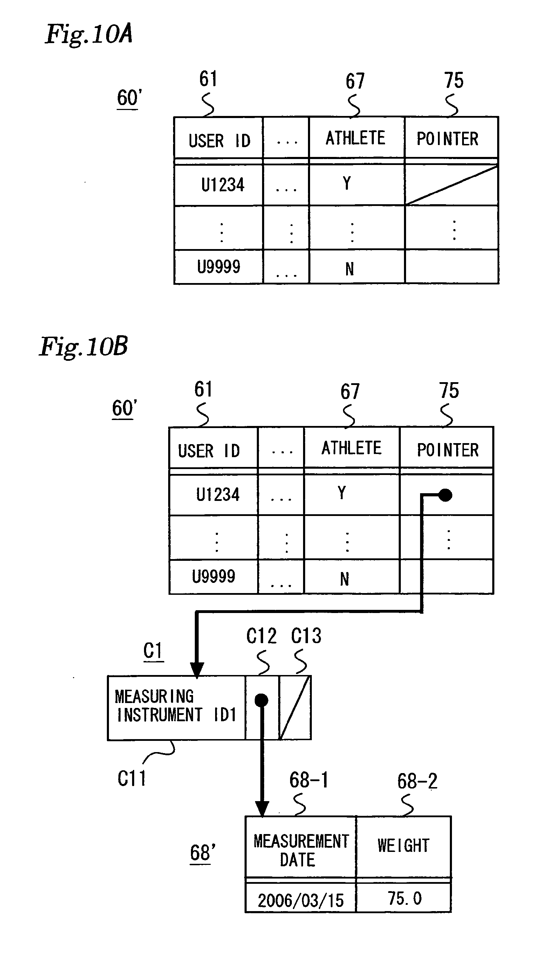

Figs. 10A and 10B show a user information table 60' or the like in a third embodiment according to the present invention. In Figs. 10A and 10B, since parts denoted by the same reference numerals as those in Fig. 2 represent the same elements, an explanation thereof will be omitted, and a password section 62 to a birthday section 66 will be omitted for the convenience's sake of the drawing. In Figs. 10A and 10B, a pointer section 75 represents a pointer to a measuring instrument (and measurement data) subjected to measuring instrument registration. As shown in Fig. 10A, since NULL (indicated by a slant line) is set in the pointer section 75 of a user having a user ID of U1234, this means that measurement using the data measuring unit 14 or the like is not performed yet. Here, when the user carries out measurement by a scale (a measuring instrument ID is a measuring instrument ID1), measurement data transmitted from the scale and received by a measurement data receiving section 24 includes the measuring instrument ID1 of the scale. Therefore, the measuring instrument ID1 is also included in measurement information transmitted by the network transmitting section 32, whereby the measurement information recording section 51 acquires the measuring instrument ID1 of the scale. Since NULL is still set in the pointer section 75 of the user, the measurement information recording section 51 determines the scale has not been subjected to measuring instrument registration in relation to the user, and subjects the measuring instrument ID1 of the scale to measuring instrument registration as a new ID. Fig. 10B shows a state where measuring instrument registration of a scale and recording of weight measurement data have been carried out in relation to the user. As shown in Fig. 10B, a pointer to a cell C1 is recorded in the pointer section 75, the measuring instrument ID1 is recorded in a left cell C11 of the cell C1, a pointer to a weight table 68' is recorded in a central cell C12 of the cell C1, and a measurement date and weight measurement data are recorded in the weight table 68'. NULL is set in a right cell C13 of the cell C1, and this means that any other data measuring instrument is not registered. Next, in case of repeating measurement by using the scale having the measurement instrument ID1, since it can be understood that the scale has been already registered by tracing the pointer section 75 to obtain contents in the cell C11, tracing the central cell C12 to record new measurement data or the like in the weight table 68' can suffice.

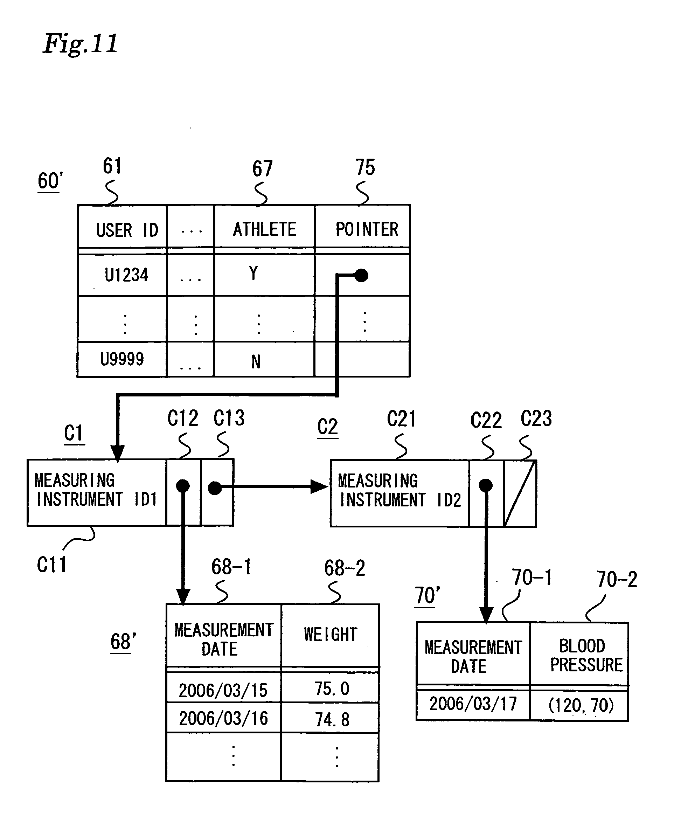

When the user newly performs measurement by using an electronic sphygmomanometer (a measuring instrument ID is a measuring instrument ID2), measurement data transmitted from the electronic sphygmomanometer 16 and received by the measurement data receiving section 24 includes the measuring instrument ID2 of the electronic sphygmomanometer 16. Therefore, measurement information transmitted by the network transmitting section 32 also includes the measuring instrument ID2, whereby the measurement information recording section 51 obtains the measuring instrument ID2 of the electronic sphygmomanometer 16. As shown in Fig. 10B, since a pointer to the cell C1 is recorded in the pointer 75 section of the user and the measuring instrument ID1 is recorded in the left cell C11 of the cell C1, the measurement information recording section 51 further obtains contents in a right cell C13. Since NULL is set in the right cell C13, the measurement information recording section 51 determines that measuring instrument registration of the electronic sphygmomanometer 16 has not been carried out in relation to the user, and subjects the measuring instrument ID2 of the electronic sphygmomanometer 16 to measuring instrument registration as a new ID. Fig. 11 shows a user information table 60' or the like when measuring instrument registration of the electronic sphygmomanometer 16 and recording of blood pressure measurement data have been performed in relation to the user. In Fig. 11, since parts denoted by the same reference numerals as those in Figs. 10A and 10B represent the same elements, thereby omitting an explanation thereof. As shown in Fig. 11, a pointer to a cell C2 is recorded in a cell C13, the measuring instrument ID2 is recorded in a left cell C21 of the cell C2, a pointer to a blood pressure table 70' is recorded in a central cell C22 of the cell C2, and measurement data or the like are recorded in a measurement date section 70-1 and a blood pressure section 70-2 in the blood pressure table 70'. NULL is set in a right cell C23 of the cell C2, and this means that a data measuring instrument other than the scale and the electronic sphygmomanometer 16 is not registered. Next, in case of repeating measurement by using the electronic sphygmomanometer 16 having the measuring instrument ID2, it can be understood that the electronic sphygmomanometer 16 has been already registered by tracing the pointer section 75 and the cell C13 to obtain contents in the cell C21. Thus, tracing the central cell C22 to record new measurement data or the like in the blood pressure table 70' can suffice. Fig. 11 shows that measurement data obtained by performing measurement for a plurality of number of times is recorded in a weight table 68'.

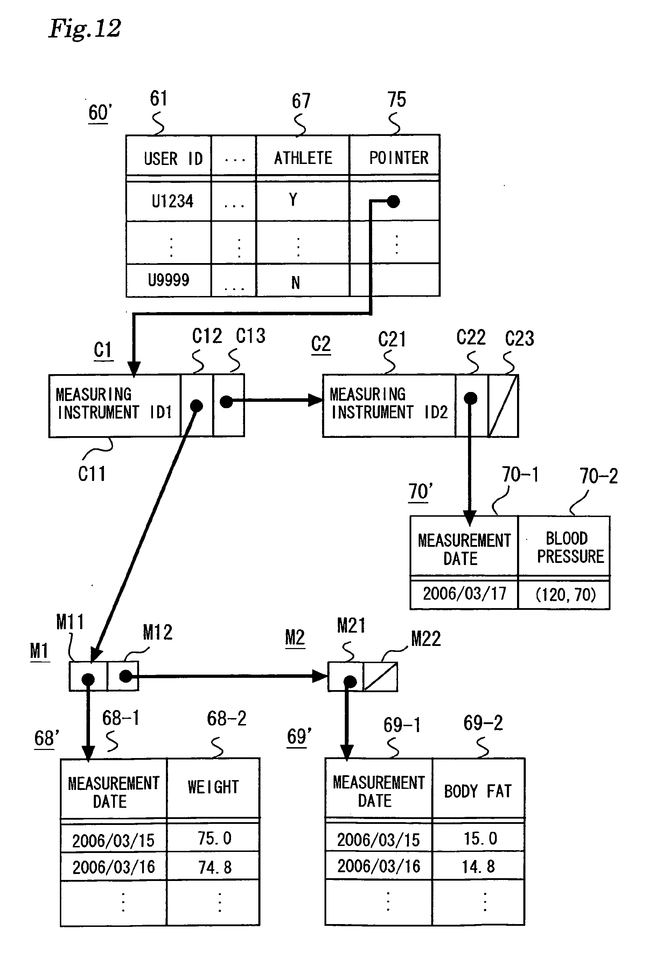

A body composition monitor 14 also measures a weight when measuring a body fat. Therefore, measurement data transmitted from a body composition monitor 14 and received by the measurement data receiving section 24 includes a measuring instrument ID3 of the body composition monitor 14 as well as two types of measurement data (a body fat percentage and a weight). Fig. 12 shows a user information table 60' or the like when a plurality of types of measurement data are included like the body composition monitor 14. In Fig. 12, since parts denoted by the same reference numerals as those in Fig. 11 represent the same elements, thereby omitting an explanation thereof. As shown in Fig. 12, a point that the measuring instrument ID3 of the body composition monitor 14 is recorded in a left cell C11 of a cell C1 is the same as those in the examples of the other data measuring instruments, but a pointer recorded in a central cell C12 points a cell M1 without directly pointing a weight table 68', and a pointer recorded in a left cell M11 of the cell M1 points the weight table 68'. A pointer recorded in a right cell M12 of the cell M1 points a cell M2, and a pointer recorded in a left M21 of a cell M2 points a body fat table 69'. Measurement data or the like are recoded in a measurement date section 69-1 and a body fat section 69-2 in the body fat table 69'. Since NULL is recorded in a right cell M22 of the cell M2, it can be understood that two types of measurement data of the body composition monitor 14 are present.

As described above, according to a third embodiment of the present invention, the number of the weight sections 68 or the like can be dynamically provided in accordance with addition or the like of the data measuring instrument. As a result, even when a user uses a new data measuring instrument, using a relay key 12 with respect to the new data measuring instrument like the existing data measuring instrument to measure data and connecting the relay key 12 with a PC 11 like as before allows a provider side server 44 to recognize use of the new data measuring instrument. A browse providing section 52 can appropriately individually use display formats 35 in accordance with types of measurement data of the new data measuring instrument. In recent years, a concept of a metabolic syndrome that visceral fat obesity causes various kinds of diseases has been considered, and a diagnosis based on a plurality of types of measurement data is required. According to a third embodiment of the present invention, it is possible to provide the data management system that can solve a problem and can not add a load to a user side by just using a desired new data measuring instrument even when such a diagnosis is required.

### Industrial Applicability

As an example of exploiting the present invention, there is an application to a health care system when a user is a person who does not have time to input measurement data to a PC by himself/herself owing to pressure of business, e.g., a company worker, or a person who is unaccustomed to and hard to operate a PC or the like, e.g., an aged person in a solitary house where he/she lives alone.

According to the data management system or the like of the present invention, when a user pushes a communication button of a relay key, predetermined information is transmitted to a data measuring instrument. The data measuring instrument is automatically activated based on the predetermined information to measure data, e.g., a weight and transmit the obtained measurement data to the relay key side. The relay key receives the measurement data transmitted from the data measuring instrument that is activated based on the predetermined information transmitted to the data measuring instrument and has performed measurement. That is, just pushing the communication button can acquire the measurement data. The relay key transmits measurement information based on the received measurement data to a PC. It is preferable that the data measuring instrument and the relay key perform communication with each other based on a wireless communication system using infrared rays or specified low power radio or the like and the relay key and the PC are connected through an interface using a USB, and hence the measurement data obtained by measurement in the data measuring instrument can be automatically transmitted to the PC through the relay key in real time. Therefore, a measurement target person does not have to take trouble in inputting the measurement data. As a configuration in a home system, the PC, the relay key, and the data measuring instrument alone can suffice, thereby providing an inexpensive device configuration. The PC transmits the measurement information transmitted from the relay key to a server on a health care provider side. The server records the transmitted measurement information in a database DB based on a predetermined format, and allows the PC side to browse the measurement information recorded in the database DB. A series of operations, i.e., transmission of the measurement information from the PC to the server, recording of the measurement information in the database DB, and provision of browsing the measurement information to the PC can be automatically performed in a state where the relay key is connected with a USB port of the PC. As a result, it is possible to advantageously provide the data management system or the like by which a distance between the data measuring instrument, e.g., a body composition monitor and the PC is not restricted to a length of the wire cable, a troublesome operation, i.e., attachment/detachment of a memory medium for data exchange is not required, inputting the measurement data, e.g., health testing data does not take trouble, and a device configuration is inexpensive.

In the data management system, the data management server may further comprise browse providing means for allowing the information terminal device side to browse the measurement information recorded in the data recording section.

In the data management system, the data management server may further comprise: judgment means for judging whether the data transmitter-receiver has been registered before use based on user information including user-authenticating information previously recorded in the data recording section and device authenticating information of the data transmitter-receiver acquired through the information terminal device; user authenticating information acquiring means for acquiring the user authenticating information through the information terminal device when the judging means determines that the data transmitter-receiver has not been registered before use; searching means for searching the data recording section based on the user authenticating information acquired from the user authenticating information acquiring means; associating means for associating the user authenticating information searched by the searching means with the device authenticating information; and registering means for registering user information corresponding to the user authenticating information searched by the searching means and excluding the user authenticating information in the data transmitter-receiver side through the information terminal device.

In the data management system, the measurement information transmitted by the measurement information transmitting means may include the device authenticating information of the data transmitter-receiver, and the measurement information recording means may record the measurement information in the data recording section in accordance with the user authenticating information based on a predetermined format when the device authenticating information included in the measurement information transmitted by the network transmitting means is associated with the user authenticating information.

In the data management system, the browse providing means request the information terminal device side to input user authenticating information, and allows the information terminal device side to browse the measurement information corresponding to the user authenticating information when the input user authenticating information matches with the user authenticating information recorded in the data recording section.

In the data management system, the user authenticating information may be one or more combinations of a user identifier, a password, and physical characteristics of a user.

In the data management system, the measurement data received by the measurement data receiving means and transmitted from the data measuring instrument may include measuring instrument identifying information of the data measuring instrument, the measuring instrument identifying information used in accordance with each user being registered in the data recording section, and the measurement information recording means may register the measuring instrument identifying information and records the measurement information in the data recording section in accordance with each user based on a predetermined format when it is determined that the measuring instrument identifying information of the data measuring instrument included in the measurement information transmitted by the network transmitting means has not been registered in relation to a user corresponding to the measurement information.

In the data transmitter-receiver, the measurement information may be transmitted from the information terminal device to a data management server connected therewith through a network, recorded in a data recording section on the data management server side in accordance with each user based on a predetermined format, and allowed to be browsed on the information terminal device side by the data management server.

In the data transmitter-receiver, the data management server may determine that the data transmitter-receiver has not been registered before use based on user information including user authenticating information previously recorded in the data recording section and device authenticating information of the data transmitter-receiver acquired through the information terminal device, the data recording section may be searched based on the user authenticating information acquired through the information terminal device, the searched user authenticating information may be associated with the device authenticating information, and user information corresponding to the searched user authenticating information and excluding the user authenticating information is registered in the data transmitter-receiver side through the information terminal device.

In the data transmitter-receiver, the measurement information transmitted by the measurement information transmitting means may include the device authenticating information of the data transmitter-receiver, and the data management server may record the measurement information in the data recording section in accordance with the user authenticating information based on a predetermined format when the device authenticating information included in the measurement information is associated with the user authenticating information.

In the data transmitter-receiver, when the data management server may request the information terminal device side to input user authenticating information and the input user authenticating information may match with the user authenticating information recorded in the data recording section, the measurement information corresponding to the user authenticating information may be allowed to be browsed on the information terminal device side.

In the data transmitter-receiver, the user authenticating information may be one or more combinations of a user identifier, a password, and physical characteristics of a user.

In the data transmitter-receiver, the measurement data received by the measurement data receiving means and transmitted from the data measuring instrument may include measuring instrument identifying information of the data measuring instrument, the measuring instrument identifying information used in accordance with each user being registered in the data recording section, and the data management server may register the measuring instrument identifying information and records the measurement information in the data recording section in accordance with each user based on a predetermined format when it is determined that the measuring instrument identifying information of the data measuring instrument included in the measurement information transmitted from the information terminal device has not been registered in relation to a user corresponding to the measurement information.

Here, the data management may further comprise browse providing means for allowing the information terminal device side to browse the measurement information recorded in the data recording section.

Here, the data management server may further comprise: judging means for judging whether the data transmitter-receiver has been registered before use based on user information including user authenticating information previously recorded in the data recording section and device authenticating information of the data transmitter-receiver acquired through the information terminal device; user authenticating information acquiring means for acquiring the user authenticating information through the information terminal device when the judging means determines that the data transmitter-receiver has not been registered before use; searching means for searching the data recording section based on the user authenticating information acquired by the user authenticating information acquiring means; associating means for associating the user authenticating information searched by the searching means with the device authenticating information; and registering means for registering the user information corresponding to the user authenticating information searched by the searching means and excluding the user authenticating information in the data transmitter-receiver side through the information terminal device.

In the data management server, the measurement information transmitted through the information terminal device may include the device authenticating information of the data transmitter-receiver, and the measurement information recording means records the measurement information in the data recording section in accordance with the user authenticating information based on a predetermined format when the device authenticating information is associated with the user authenticating information.

In the data management server, the browse providing means may request the information terminal device side to input user authenticating information, and may allow the information terminal device side to browse the measurement information corresponding to the user authenticating information when the input user authenticating information matches with the user authenticating information recorded in the data recording section.

In the data management server, the user authenticating information may be one or more combinations of a user identifier, a password, and physical characteristics of a user.

In the data management server, the measurement data transmitted from the data measuring instrument may include measuring instrument identifying information of the data measuring instrument, the measuring instrument identifying information used in accordance with each user being registered in the data recording section, and the measurement information recording means may register the measuring instrument identifying information and records the measurement information in the data recording section in accordance with each user based on a predetermined format when it is determined that the measuring instrument identifying information of the data measuring instrument included in the measurement information transmitted through the information terminal device has not been registered in relation to a user corresponding to the measurement information.

Here, the data management method may further comprise a browse providing step at which the data management server allows the information terminal device side to browse the measurement information recorded in the data recording section.

Here, the data management method may further comprise: a judging step at which the data management server judges whether the data transmitter-receiver has been registered before use based on user information including user authenticating information previously recorded in the data recording section and device authenticating information of the data transmitter-receiver acquired through the information terminal device; a user authenticating information acquiring step at which the data management server acquires the user authenticating information through the information terminal device when it is determined that the data transmitter-receiver has not been registered before use in the judging step; a searching step at which the data management server searches the data recording section based on the user authenticating information acquired in the user authenticating information acquiring step; an associating step at which the data management server associates the user authenticating information searched in the searching step with the device authenticating information; and a registering step at which the data management server registers the user information corresponding to the user authenticating information searched in the searching step and excluding the user authenticating information in the data transmitter-receiver side through the information terminal device.

In the data management method, the measurement data received in the measurement data receiving step and transmitted from the data measuring instrument may include measuring instrument identifying information of the data measuring instrument, the measuring instrument identifying information used in accordance with each user being registered in the data recording section, and the measuring instrument identifying information may be registered and the measurement information is recorded in the data recording section in accordance with each user based on a predetermined format in the measurement information recording step when it is determined that the measuring instrument identifying information of the data measuring instrument included in the measurement information transmitted in the network transmitting step has not been registered in relation to a user corresponding to the measurement information.

The present invention has been described in detail with respect to various embodiments, and it will now be apparent from the foregoing to those skilled in the art that changes and modifications may be made without departing from the invention in its broader aspects, and it is the invention, therefore, in the appended claims to cover all such changes and modifications as fall within the true spirit of the invention.

The entire disclosure of Japanese Patent Application No. 2006-75764 filed on March 19, 2006 including specification, claims, drawings and summary are incorporated herein by reference in its entirety.

## Claims

1. A data management system **characterized by**: an information terminal device and a data management server that are connected with each other through a network; a data transmitter-receiver detachably connected with the information terminal device through a predetermined interface; and a data measuring instrument connected with the data transmitter-receiver based on a predetermined wireless communication system, wherein
said data transmitter-receiver comprises:
information transmitting means for transmitting predetermined information to said data measuring instrument;
measurement data receiving means for receiving measurement data of a user transmitted from said data measuring instrument based on predetermined information transmitted by said information transmitting means; and
measurement information transmitting means for transmitting measurement information based on the measurement data received by said measurement data receiving means to said information terminal device,
said information terminal device comprises:
network transmitting means for transmitting the measurement information transmitted by said measurement information transmitting means to said data management server, and
said data management server comprises:
measurement information recording means for recording the measurement information transmitted by said network transmitting means in a data recording section in accordance with each user based on a predetermined format.

2. The data management system according to claim 1, wherein said data management server further comprises browse providing means for allowing said information terminal device side to browse the measurement information recorded in said data recording section.

3. The data management system according to claim 1 or 2, wherein said data management server further comprises:
judgment means for judging whether said data transmitter-receiver has been registered before use based on user information including user-authenticating information previously recorded in said data recording section and device authenticating information of said data transmitter-receiver acquired through said information terminal device;
user authenticating information acquiring means for acquiring the user authenticating information through said information terminal device when said judging means determines that said data transmitter-receiver has not been registered before use;
searching means for searching said data recording section based on the user authenticating information acquired from said user authenticating information acquiring means;
associating means for associating the user authenticating information searched by said searching means with the device authenticating information; and
registering means for registering user information corresponding to the user authenticating information searched by said searching means and excluding the user authenticating information in said data transmitter-receiver side through said information terminal device.

4. The data management system according to claim 3, wherein the measurement information transmitted by said measurement information transmitting means includes the device authenticating information of said data transmitter-receiver, and
said measurement information recording means records the measurement information in said data recording section in accordance with the user authenticating information based on a predetermined format when the device authenticating information included in the measurement information transmitted by said network transmitting means is associated with the user authenticating information.

5. The data management system according to claim 4, wherein said browse providing means requests said information terminal device side to input user authenticating information, and allows said information terminal device side to browse the measurement information corresponding to the user authenticating information when the input user authenticating information matches with the user authenticating information recorded in said data recording section.

6. The data management system according to any one of claims 3-5, wherein said user authenticating information is one or more combinations of a user identifier, a password, and physical characteristics of a user.

7. The data management system according to any one of claims 1-6, wherein the measurement data received by said measurement data receiving means and transmitted from said data measuring instrument includes measuring instrument identifying information of said data measuring instrument, the measuring instrument identifying information used in accordance with each user being registered in said data recording section, and
said measurement information recording means registers the measuring instrument identifying information and records the measurement information in said data recording section in accordance with each user based on a predetermined format when it is determined that the measuring instrument identifying information of said data measuring instrument included in the measurement information transmitted by said network transmitting means has not been registered in relation to a user corresponding to the measurement information.

8. A data transmitter-receiver detachably connected with an information terminal device through a predetermined interface, **characterized by**:
information transmitting means for transmitting predetermined information to a data measuring instrument connected therewith based on a predetermined wireless communication system;
measurement data receiving means for receiving measurement data of a user transmitted from said data measuring instrument based on the predetermined information transmitted by said information transmitting means; and
measurement information transmitting means for transmitting measurement information based on the measurement data received by said measurement data receiving means to said information terminal device.

9. The data transmitter-receiver according to claim 8, wherein the measurement information is transmitted from said information terminal device to a data management server connected therewith through a network, recorded in a data recording section on the data management server side in accordance with each user based on a predetermined format, and allowed to be browsed on said information terminal device side by the data management server.

10. The data transmitter-receiver according to claim 8 or 9, wherein said data management server determines that said data transmitter-receiver has not been registered before use based on user information including user authenticating information previously recorded in said data recording section and device authenticating information of said data transmitter-receiver acquired through said information terminal device, said data recording section is searched based on the user authenticating information acquired through said information terminal device, the searched user authenticating information is associated with the device authenticating information, and user information corresponding to the searched user authenticating information and excluding the user authenticating information is registered in said data transmitter-receiver side through said information terminal device.

11. The data transmitter-receiver according to claim 10, wherein the measurement information transmitted by said measurement information transmitting means includes the device authenticating information of said data transmitter-receiver, and said data management server records the measurement information in said data recording section in accordance with the user authenticating information based on a predetermined format when the device authenticating information included in the measurement information is associated with the user authenticating information.

12. The data transmitter-receiver according to claim 11, wherein, when said data management server requests said information terminal device side to input user authenticating information and the input user authenticating information matches with the user authenticating information recorded in said data recording section, the measurement information corresponding to the user authenticating information is allowed to be browsed on said information terminal device side.

13. The data transmitter-receiver according to any one of claims 10-12, wherein the user authenticating information is one or more combinations of a user identifier, a password, and physical characteristics of a user.

14. The data transmitter-receiver according to any one of claims 8-13, wherein the measurement data received by said measurement data receiving means and transmitted from said data measuring instrument includes measuring instrument identifying information of said data measuring instrument, the measuring instrument identifying information used in accordance with each user being registered in said data recording section, and
said data management server registers the measuring instrument identifying information and records the measurement information in said data recording section in accordance with each user based on a predetermined format when it is determined that the measuring instrument identifying information of said data measuring instrument included in the measurement information transmitted from said information terminal device has not been registered in relation to a user corresponding to the measurement information.

15. A data management server connected with an information terminal device through a network,
wherein a data transmitter-receiver detachably connected with said information terminal device through a predetermined interface transmits predetermined information to a data measuring instrument connected with the data transmitter-receiver based on a predetermined wireless communication system, measurement data of a user transmitted from said data measuring instrument is received based on the predetermined information, and measurement information based on the measurement data is transmitted to said data management server through said information terminal device, and
said data management server comprises measurement information recording means for recording the transmitted measurement information in a data recording section in accordance with each user based on a predetermined format.

16. The data management server according to claim 15, further comprising browse providing means for allowing said information terminal device side to browse the measurement information recorded in said data recording section.

17. The data management server according to claim 15 or 16, further comprising:
judging means for judging whether said data transmitter-receiver has been registered before use based on user information including user authenticating information previously recorded in said data recording section and device authenticating information of said data transmitter-receiver acquired through said information terminal device;
user authenticating information acquiring means for acquiring the user authenticating information through said information terminal device when said judging means determines that said data transmitter-receiver has not been registered before use;
searching means for searching said data recording section based on the user authenticating information acquired by said user authenticating information acquiring means;
associating means for associating the user authenticating information searched by said searching means with the device authenticating information; and
registering means for registering the user information corresponding to the user authenticating information searched by said searching means and excluding the user authenticating information in said data transmitter-receiver side through said information terminal device.

18. The data management server according to claim 17, wherein the measurement information transmitted through said information terminal device includes the device authenticating information of said data transmitter-receiver, and
said measurement information recording means records the measurement information in said data recording section in accordance with the user authenticating information based on a predetermined format when the device authenticating information is associated with the user authenticating information.

19. The data management server according to claim 18, wherein, said browse providing means requests said information terminal device side to input user authenticating information, and allows said information terminal device side to browse the measurement information corresponding to the user authenticating information when the input user authenticating information matches with the user authenticating information recorded in said data recording section.

20. The data management server according to any one of claims 17-19, wherein the user authenticating information is one or more combinations of a user identifier, a password, and physical characteristics of a user.

21. The data management server according to any one of claims 15-20, wherein the measurement data transmitted from said data measuring instrument includes measuring instrument identifying information of said data measuring instrument, the measuring instrument identifying information used in accordance with each user being registered in said data recording section, and
said measurement information recording means registers the measuring instrument identifying information and records the measurement information in said data recording section in accordance with each user based on a predetermined format when it is determined that the measuring instrument identifying information of said data measuring instrument included in the measurement information transmitted through said information terminal device has not been registered in relation to a user corresponding to the measurement information.

22. A data management method using: an information terminal device and a data management server connected with each other through a network; a data transmitter-receiver detachably connected with the information terminal device through a predetermined interface; and a data measuring instrument connected with the data transmitter-receiver based on a predetermined wireless communication system, **characterized by**:
an information-transmitting step at which the data transmitter-receiver transmits predetermined information to said data measuring instrument;
a measurement data receiving step at which the data transmitter-receiver receives measurement data of a user transmitted from said data measuring instrument based on the predetermined information transmitted in said information transmitting step;
a measurement information transmitting step at which the data transmitter-receiver transmits measurement information based on the measurement data received in said measurement data receiving step to the information terminal device;
a network transmitting step at which the information terminal device transmits the measurement information transmitted in said measurement information transmitting step to the data management server, and
a measurement information recording step at which the data management server records the measurement information transmitted in said network transmitting step in a data recording section in accordance with each user based on a predetermined format.

23. The data management method according to claim 22, further comprises a browse providing step at which the data management server allows the information terminal device side to browse the measurement information recorded in the data recording section.

24. The data management method according to claim 22 or 23, further comprises:
a judging step at which the data management server judges whether the data transmitter-receiver has been registered before use based on user information including user authenticating information previously recorded in the data recording section and device authenticating information of the data transmitter-receiver acquired through the information terminal device;
a user authenticating information acquiring step at which the data management server acquires the user authenticating information through the information terminal device when it is determined that the data transmitter-receiver has not been registered before use in said judging step;
a searching step at which the data management server searches the data recording section based on the user authenticating information acquired in said user authenticating information acquiring step;
an associating step at which the data management server associates the user authenticating information searched in said searching step with the device authenticating information; and
a registering step at which the data management server registers the user information corresponding to the user authenticating information searched in said searching step and excluding the user authenticating information in the data transmitter-receiver side through the information terminal device.

25. The data management method according to any one of claims 22-24, wherein the measurement data received in said measurement data receiving step and transmitted from said data measuring instrument includes measuring instrument identifying information of said data measuring instrument, the measuring instrument identifying information used in accordance with each user being registered in the data recording section, and
the measuring instrument identifying information is registered and the measurement information is recorded in the data recording section in accordance with each user based on a predetermined format in said measurement information recording step when it is determined that the measuring instrument identifying information of said data measuring instrument included in the measurement information transmitted in said network transmitting step has not been registered in relation to a user corresponding to the measurement information.
